# EUROPEAN PATENT SPECIFICATION

(11) **EP 0 385 909 B1**
(45) Date of publication and mention of the grant of the patent: **13.07.1994**
(21) Application number: 90610014.4
(22) Date of filing: 01.03.1990
(51) Int. Cl.: A61K 39/21, A61K 37/02

(54) **A kit or composition for the prevention or treatment of HIV-1 infections**
Kit oder Zusammensetzung zur Vorbeugung oder Behandlung von HIV-1 Infektionen
Kit ou composition pour la prévention ou le traitement des infections par HIV-1

(30) Priority: 03.03.1989 US 318272
(43) Date of publication of application: 05.09.1990
(73) Proprietor: MicroGeneSys, Inc., West Haven Connecticut 06516 (US)
(72) Inventor: Volvovitz, Franklin, New Haven, CT 06511 (US)
(74) Representative: Mathiesen, Hans Preben

(56) References cited:
- EP-A- 280 468
- EP-A- 0 265 785
- EP-A- 0 272 858
- EP-A- 0 287 226
- WO-A-87/06260
- DERWENT ABSTRACT, Derwent Publications Ltd., London (GB); no. 88292709 (41)#
- VACCINE, vol. 5, no. 2, June 1987; P.J. BARR et al., pp. 90-101#
- THE LANCET, vol. II, no. 8467, 07 November 1987, London (GB); A.G. DALGLEISH et al., pp. 1047-1050#
- CHEMICAL ABSTRACTS, vol. 112, no. 17, 23 April 1990, Columbus, OH (US); p. 573, no. 156570c#
- DERWENT ABSTRACTS, Derwent Publications Ltd., London (GB); no. 89118256 (16)#
- AIDS: Papers from Science, 1982-1985, 1986; R. KULSTAD, pp. 461-483#
- TREATMENT ISSUES, vol. 2, no. 7, 20 October 1988; A. MANIKER, pp. 1-12#
- CRC, vol. 8, no. 4, 1988; W.L. FARRAR, pp. 315-339#
- NEW ENGLAND JOURNAL OF MEDICINE, vol. 324, 13 June 1991, pp. 1677-1684#

## Description

This invention relates to a kit or composition for the prevention and/or treatment of AIDS or HIV-1 infections. At present there is no known effective treatment for HIV-1 infections in humans.

Much research work has been carried out to develope not only an understanding of the human immunodeficiency virus, HIV-1, the apparent causative agent for AIDS or HIV-1 infections, but also to develop chemotherapeutic agents or pharmaceutical products for the treatment and/or cure of HIV-1 infections. As yet, no chemotherapeutic agent or pharmaceutical product has been developed which is satisfactory for the treatment of HIV-1 infections. Attention has also been directed to the use of immunotherapy for the treatment and cure of HIV-1 infections. Here also success has not yet been achieved.

It is an object of this invention to provide materials and methods of employing the same for preparing a kit or composition useful for the prevention and/or treatment of HIV infections.

It is another object of this invention to provide materials and method of employing the same for preparing a kit or composition comprising (1) CD4 and/or its derivatives and (2) an immunogenic HIV-1 protein which can readily be administered to humans, including neonates who may have received the HIV-1 infection from their mothers, and adults who have been exposed to HIV-1 infection or who are infected with HIV-1.

How these and other objects of this invention are achieved will become apparent in the light of the accompanying disclosure. In at least one embodiment of the practices of this invention at least one of the foregoing objects will be achieved.

It has now been discovered that a special combination of agents, viz. CD4, particularly soluble CD4, and its derivatives, and immunogenic HIV-1 protein or vaccine material, when administered to a human exposed to or suffering from HIV-1 infection provides a useful treatment for the prevention and/or therapy for AIDS or HIV-1 infections in humans.

In the practices of this invention an HIV-1 vaccine material, such as immunogenic HIV-1 envelope protein, e.g. gp160, is used in conjunction or in association with CD4, preferably soluble CD4, a cell surface glycoprotein found mostly on a subset of mature peripheral T cells that recognize antigens presented by class II MHC molecules. HIV-1 envelope protein, such as gp120 and gp160, binds CD4 with high affinity.

In the use of these materials in accordance with this invention, CD4 and immunogenic HIV-1 protein, CD4 is preferably first administered to the human suffering from HIV-1 infection or for its prevention. The administration of the CD4 is usually preferably followed by the administration of a suitable HIV-1 vaccine, such as immunogenic HIV-1 protein, such as gp160 and gp120, both HIV-1 envelope proteins, or p24 HIV-1 core protein, or p66 HIV-1 reverse transcriptase protein. These immunogenic protein materials derived from HIV-1 can be employed singly or in combination. Of these materials gp160 provides excellent results in providing active immunization which is advantageously used or employed by passive treatment with the CD4.

The administration of CD4 to a human suffering from HIV-1 infection fairly promptly reduces the HIV-1 virus load and the immunization with the HIV-1 protein, such as gp160, elicits a strong immune response, including a strong cellular component against both free HIV-1 virus and virus infected cells. The use of CD4, particularly soluble CD4, enhances the therapeutic value of the companion administered HIV-1 vaccine material, such as the administered immunogenic HIV-1 protein, e.g. gp160. Inversely, the use of the active immunization by means of the immunogenic HIV-1 protein vaccine enhances the therapeutic value of the administered CD4 since CD4 binds with free virus and, as indicated, reduces virus load and additionally also binds with virus infected cells, thereby increasing the likelihood these cells will be eliminated through cellular immune mechanisms.

In the practices of this invention recombinant immunogenic HIV-1 proteins are usefully employed as are recombinant CD4, particularly soluble CD4, and their derivtives. The preparation of therapeutic materials by recombinant DNA or genetic engineering techniques is well established and well known, particularly since the pioneering work and discoveries described in the Cohen and Boyer patents U.S. 4,237,224 and 4,468,464. Recombinant DNA techniques employing recombinant baculovirus vectors for the expression of foreign genes in insect cells, are well established, see the disclosures of the following European Patent Applications No. 0127839 published December 12, 1984, No 0155476 published September 25, 1988, No. 0175852 published April 2, 1986, No. 0228036 published July 8, 1987, No. 0265785 published May 4, 1988, No. 0272858 published June 29, 1988, No. 0279688 published August 24, 1988, and No. 0273366 published July 6, 1986. U.S. Patent 4,745,057 corresponds to the aforementioned published European patent application No. 0127839.

In the practices of this invention it is preferred to employ recombinant produced CD4 materials or derivatives and recombinant produced immunogenic HIV-1 protein materials, such as p24, p66, gp120 and gp160. In this connection, see also EP-A-228 036, EP-A-265 785, EP-A-288 093 and EP-A-327 180. Although recombinant insect cell expressed materials are preferred in the practices of this invention, recombinant materials expressed in other hosts, such as other mammalian cells and bacterial cells, including yeast cells, as well as materials derived from human sources are also usefully employed in the practices of this invention, see U.S. Patent 4,725,669 which discloses the recovery of gp120 and gp160 from human cells.

In addition to the patent literature, the reseach literature is also rich with respect to matters relating to the treatment of HIV-1 infections, including CD4 materials and immunogenic HIV-1 envelope protein materials. In this connection attention is directed to Bio/Technology, Vol. 4, September 1986, pp. 790-795; and the research papers reported in Proc. Natl. Acad. Sci. U.S.A., Vol. 84, pp. 4601-4605, July 1987; Nature, No. 334, No. 6178 pp. 159-162, July 14, 1988, Proc. Natl. Acad. Sci., Vol. 85, pp. 5190-5994, July 1988, Proc. Natl. Acad. Sci., Vol. 85, pp. 9273-9277, December 1988.

Although the administration of the materials in accordance with this invention for the prevention or treatment of HIV-1 infections is preferably carried out by intramuscular injection, these materials may be administered subcutaneously, particularly to a person of high risk of hemorrhage following intramuscular injection. The materials may be administered at substantially the same time but at different locations, such as one injection of CD4 into the thigh, followed by the injection of the HIV-1 vaccine material, e.g. gp160, into the opposite anterolateral thigh. This technique is preferable so as to ensure better absorption of the administered HIV-1 immunogenic vaccine material.

As indicated hereinabove, it is usually preferred to administer the CD4 first but, if desired, the HIV-1 immunogenic vaccine material may be administered first. A preferred routine for the administration of the materials in accordance with this invention for the prevention and/or treatment of HIV-1 infections would involve first the administration of the CD4 component, such as about one 0.5 ml dose, followed by the administration of three separate 10 x 10⁻⁶g (mcg) 1.0 ml doses of the HIV-1 vaccine material, the three doses being administered over a period of time, such as the first dose administered 7 days after the administration of the CD4, the second dose being administered about one month thereafter and the third being administered about six months thereafter. If desired, these doses may be increased in number and the period of time over which the doses are administered may be increased, such as up to about one year, and the dosages may be reduced. Information indicates that reduced total dosages of the HIV-1 vaccine material three separate doses of 10 x 10⁻⁶g (mcg) per 0.5 ml are about equally as effective as the larger 20 x 10⁻⁶g (mcg) 0.5 ml doses.

For an infant or neonate born of an HIV-1 positive mother it is recommended that at birth 0.5 ml of CD4, preferably soluble CD4, be administered, followed by the administration of three separate doses of the HIV-1 vaccine material, one dose administered about 7 days after birth in the amount 10-30 x 10⁻⁶g (mcg) 0.5 ml, the second dose administered one month after birth in the same amount and the third dose being administered six months after birth, also in the same amount. As indicated hereinabove, for a person who has been exposed to the HIV-1 virus, such as through a percutaneous, ocular or mucous membrane exposure to blood known to contain HIV-1, the CD4 is promptly administered, such as in an amount 0.06 ml/kg, preferably within 24 hours after exposure, followed by three separate administrations of the HIV-1 vaccine material, one administration 7 days after exposure and the second and third administrations being given during the one to six month period after exposure, or over a longer period of time, if desirable.

## Claims

1. A kit make-up or composition useful for the prevention or treatment of HIV-1 infections comprising (1) CD4 and/or its derivatives and (2) an immunogenic HIV-1 protein.

2. A composition in accordance with Claim 1 wherein said HIV-1 protein is recombinant protein expressed in an insect cell.

3. A composition in accordance with Claim 1 wherein said CD4 derivative is a CD4- immunoglobulin hybrid or complex.

4. A composition in accordance with Claim 1 wherein the protein and CD4 components are maintained in said kit in separate containers.

5. A kit useful for the prevention or treatment of HIV-1 infections comprising (1) CD4 and/or its derivatives and (2) an immunogenic HIV-1 protein and means for administering the same to a human.

6. The use of (1) CD4 and/or its derivatives and (2) an immunogenic HIV-1 protein for preparing a kit make-up or composition useful for the prevention or treatment of HIV-1 infections in humans.

7. The use in accordance with Claim 6 wherein in addition to CD4 and/or its derivatives the HIV-1 protein is incorporated into the kit or composition in multiple doses to be administered over a period of time up to about 12 months.

## Patentansprüche

1. Kit oder Zusammensetzung, welche(r) geeignet ist zur Prävention oder Behandlung von HIV-1-Infektionen, umfassend (1) CD4 und/oder dessen Derivate und (2) ein immunogenes HIV-1-Protein.

2. Zusammensetzung nach Anspruch 1, bei der das HIV-1-Protein ein von einer Insektenzelle exprimiertes rekombinantes Protein ist.

3. Zusammensetzung nach Anspruch 1, bei der das CD4-Derivat ein CD4-Immunglobulinhybrid oder -komplex ist.

4. Zusammensetzung nach Anspruch 1, bei der die Protein- und CD4-Komponenten in dem Kit in getrennten Behältern bereitgehalten werden.

5. Kit, welcher zur Prävention oder Behandlung von HIV-1-Infektionen geeignet ist, umfassend (1) CD4 und/oder dessen Derivate und (2) ein immunogenes HIV-1-Protein und Mittel zur Verabreichung derselben an einen Menschen.

6. Verwendung von (1) CD4 und/oder dessen Derivaten und (2) eines immunogenen HIV-1-Proteins zur Herstellung eines Kits oder einer Zusammensetzung, die zur Prävention oder Behandlung von HIV-1-Infektionen bei Menschen geeignet sind.

7. Verwendung nach Anspruch 6, bei der das HIV-1-Protein zusätzlich zu CD4 und/oder dessen Derivaten in dem Kit oder der Zusammensetzung in multiplen Dosierungen eingeschlossen ist, um über eine Zeitdauer von bis zu etwa 12 Monaten verabreicht zu werden.

## Revendications

1. Préparation en kit ou composition utile pour la prévention ou le traitement d'infections à HIV-1 comprenant (1) du CD4 et/ou ses dérivés et (2) une protéine de HIV-1 immunogène.

2. Composition selon la revendication 1 dans laquelle la protéine de HIV-1 est une protéine recombinante exprimée dans une cellule d'insecte.

3. Composition selon la revendication 1 dans laquelle le dérivé de CD4 est un hybride ou un complexe CD4-immunoglobuline.

4. Composition selon la revendication 1 dans laquelle la protéine et les composants de CD4 sont conservés dans ledit kit dans des récipients différents.

5. Kit utile pour la prévention ou le traitement d'infections à HIV-1 comprenant (1) du CD4 et/ou ses dérivés et (2) une protéine de HIV-1 immunogène et des moyens pour l'administration de ceux-ci à un être humain.

6. Utilisation de (1) CD4 et/ou ses dérivés et (2) une protéine de HIV-1 immunogène pour l'obtention d'une préparation en kit ou d'une composition utile pour la prévention ou le traitement d'infections à HIV-1 chez des êtres humains.

7. Utilisation selon la revendication 6 dans laquelle on incorpore dans le kit ou la composition, en plus du CD4 et/ou ses dérivés, la protéine de HIV-1 selon des doses multiples destinées à être administrées sur une période pouvant aller jusqu'à environ 12 mois.
